(19) Europäisches Patentamt
European Patent Office
Office européen des brevets

(11) **EP 2 736 558 B1**

(12) **EUROPÄISCHE PATENTSCHRIFT**

(45) Veröffentlichungstag und Bekanntmachung des Hinweises auf die Patenterteilung:
**20.09.2017   Patentblatt 2017/38**

(21) Anmeldenummer: **12743675.6**

(22) Anmeldetag: **27.07.2012**

(51) Int Cl.:
***A61M 1/34*** *(2006.01)*

(86) Internationale Anmeldenummer:
**PCT/EP2012/003190**

(87) Internationale Veröffentlichungsnummer:
**WO 2013/017236 (07.02.2013 Gazette 2013/06)**

(54) **VERFAHREN SOWIE VORRICHTUNGEN ZUM ÜBERPRÜFEN WENIGSTENS EINER FUNKTION EINER MEDIZINISCHEN FUNKTIONSEINRICHTUNG**

METHOD AND DEVICES FOR TESTING AT LEAST ONE FUNCTION OF A MEDICAL FUNCTIONAL DEVICE

PROCÉDÉ ET DISPOSITIFS DE CONTRÔLE D'AU MOINS UNE FONCTION D'UN DISPOSITIF FONCTIONNEL MÉDICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.07.2011   DE 102011108784**
**29.07.2011   US 201161512984 P**

(43) Veröffentlichungstag der Anmeldung:
**04.06.2014   Patentblatt 2014/23**

(73) Patentinhaber: **Fresenius Medical Care Deutschland GmbH**
**61352 Bad Homburg (DE)**

(72) Erfinder:
• **GRONAU, Soeren**
**65428 Rüsselsheim (DE)**
• **MANKE, Joachim**
**35792 Loehnberg (DE)**
• **MUELLER, Ralf**
**61350 Bad Homburg (DE)**
• **SCHAEFER, Ingo**
**65760 Eschborn (DE)**

(74) Vertreter: **Bobbert & Partner Patentanwälte PartmbB Postfach 1252 85422 Erding (DE)**

(56) Entgegenhaltungen:
EP-A1- 1 319 417          EP-A1- 1 595 560
EP-A1- 2 174 677          DE-A1-102007 024 463
DE-A1-102007 042 964      DE-C1- 19 757 523
US-A1- 2004 223 857       US-A1- 2005 126 998
US-A1- 2010 192 686

EP 2 736 558 B1

**Beschreibung**

Die vorliegende Erfindung betrifft ein Verfahren gemäß Anspruch 1. Sie betrifft zudem eine Erfassungseinrichtung gemäß Anspruch 8 und eine medizinische Behandlungsvorrichtung gemäß Anspruch 12.

Aus der Praxis sind maschinelle Tests zum Überprüfen von Funktion und Zuverlässigkeit von medizinischen Funktionseinrichtungen wie extrakorporalen Blutschläuchen, Blutkassetten und dergleichen bekannt. Ein solcher Test ist als Druckhaltetest bekannt. Er wird durchgeführt nachdem die in Frage stehende Funktionseinrichtung mit der Behandlungsvorrichtung verbunden ist, also nachdem die Behandlungsvorrichtung bereits mit der Funktionseinrichtung aufgerüstet ist, und bevor die Behandlung des Patienten beginnt.

In der Patentanmeldung US 2004/0223857 A1 wird ein Verarbeitungssystem für Blutbestandteile beschrieben, das eine Kassette und eine Steuerung umfasst. Die Steuerung überprüft die Installation und Integrität der Kassette.

Die Patentanmeldung DE 102207 042 964 A1 beschreibt ein Verfahren zur Überprüfung der Dichtigkeit einer medizinischen Kassette mit flüssigkeitsführenden Kanälen, welche von einer flexiblen Folie abgedeckt sind. Durch die Möglichkeit, kontinuierlich Luft zwischen der flexiblen Folie und einer Ankoppelfläche abzusaugen, ergibt sich die Möglichkeit, Undichtigkeiten der Folie festzustellen.

Eine Aufgabe der vorliegenden Erfindung ist es, ein weiteres Verfahren zur Überprüfung der Funktion von medizinischen Funktionseinrichtungen vorzuschlagen. Zudem sollen geeignete Vorrichtungen, ein geeignetes digitales Speichermedium, ein geeignetes Computerprogramm-Produkt und ein geeignetes Computerprogramm angegeben werden.

Die erfindungsgemäße Aufgabe wird durch ein Verfahren mit den Merkmalen des Anspruchs 1 gelöst. Sie wird ferner gelöst mittels der Erfassungseinrichtung mit den Merkmalen des Anspruchs 8 und der Behandlungsvorrichtung mit den Merkmalen des Anspruchs 12.

Erfindungsgemäß wird somit ein Verfahren vorgeschlagen zum Überprüfen wenigstens einer Funktion einer medizinischen Funktionseinrichtung, während diese in eine medizinische Behandlungsvorrichtung eingelegt und mit dieser, insbesondere in Fluidkommunikation, verbunden ist, und/oder zum Überprüfen einer Funktion der mit der Funktionseinrichtung verbundenen Behandlungsvorrichtung.

Das Verfahren kann ein Einlegen der Funktionseinrichtung in die medizinische Behandlungsvorrichtung umfassen oder es setzt dieses wenigstens voraus.

Weiter kann das Verfahren das Verpressen, das Vorspannen oder das Einklemmen der Funktionseinrichtung in der Behandlungsvorrichtung, beispielsweise mittels der Tür der Behandlungsvorrichtung, umfassen. Alternativ setzt es dies voraus.

Weiter kann das Verfahren das Herstellen wenigstens einer Fluidkommunikation zwischen einer Hydraulikeinrichtung oder einer Pneumatikeinheit der Behandlungsvorrichtung und der Funktionseinrichtung umfassen. Alternativ liegt dieser Zustand bereits vor Beginn des Verfahrensablaufs vor.

Das erfindungsgemäße Verfahren umfasst das Aufbauen eines Drucks mittels der Hydraulikeinrichtung oder einer Pneumatikeinheit innerhalb der Fluidkommunikation. Es umfasst zudem ein sich hieran anschließendes Messen eines nach Aufbauen des Drucks innerhalb der Fluidkommunikation herrschenden Drucks oder einer Druckveränderung wie eines Druckanstiegs oder eines Druckabfalls.

Ferner umfasst das erfindungsgemäße Verfahren ein Treffen einer Aussage über die überprüfte Funktion der Funktionseinrichtung aufgrund eines Vergleichs des herrschenden Drucks oder der gemessenen Druckänderung mit zuvor gespeicherten Werten, Schwellenwerten, Bereichen oder Verläufen.

Die erfindungsgemäße Erfassungseinrichtung ist geeignet und vorgesehen und/oder ausgelegt und/oder konfiguriert zur Durchführung des erfindungsgemäßen Verfahrens.

Die erfindungsgemäße medizinische oder medizintechnische Behandlungsvorrichtung (im Folgenden auch kurz: Behandlungsvorrichtung) weist wenigstens eine Erfassungseinrichtung auf und/oder ist hiermit in Signalübertragung verbunden oder steht mit dieser in einer Signalübertragungsbeziehung.

Ein erfindungsgemäßes digitales, insbesondere nichtflüchtiges, Speichermedium, insbesondere in Form eines maschinenlesbaren Trägers, insbesondere in Form einer Diskette, CD, DVD oder EPROM, insbesondere mit elektronisch oder optisch auslesbaren Steuersignalen, kann derart mit einem programmierbaren Computersystem zusammenwirken, dass die maschinellen Schritte eines erfindungsgemäßen Verfahrens veranlasst werden.

Dabei können alle, einige oder manche der maschinell durchgeführten Schritte des erfindungsgemäßen Verfahrens veranlasst werden.

Ein erfindungsgemäßes Computerprogramm-Produkt weist einen volatilen, flüchtigen oder auf einem maschinenlesbaren Träger gespeicherten Programmcode zur Veranlassung der maschinellen Schritte des erfindungsgemäßen Verfahrens, wenn das Computerprogramm-Produkt auf einem Rechner abläuft, auf. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Ein erfindungsgemäßes Computerprogramm weist einen Programmcode auf zur Veranlassung der maschinellen Schritte des erfindungsgemäßen Verfahrens, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Auch für das erfindungsgemäße Computerprogramm-Produkt und das erfindungsgemäße Computerprogramm gilt, dass alle, einige oder manche der maschinell durchgeführten Schritte des erfindungsgemäßen Verfahrens veranlasst werden.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

In manchen erfindungsgemäßen Ausführungsformen wird eine vorbestimmten Wartezeit oder Messzeit abgewartet, bevor eine Aussage über die geprüfte Funktion getroffen wird.

Ein Messen eines Drucks oder einer Druckveränderung kann jede Art von Feststellen umfassen, beispielsweise ein Messen mittels eines Sensors, ein Errechnen, ein Schließen aufgrund von Zahlenwerten anderer Parameter oder Umstände, usw.

Ebenso kann ein Festellen, wie hierin verwendet, ein Prüfen auf Vorliegen oder Nichtvorliegen, ein Messen, ein "zu einem Ergebnis oder einer Aussage komme", ein Erkennen, ein Ermitteln, oder dergleichen sein.

In manchen erfindungsgemäßen Ausführungsformen weisen die hierin beschriebenen Einrichtungen, Vorrichtungen, Apparate und andere Gegenstände die zum Ausführen von hierin beschriebenen Verfahrensschritten oder Testschritten erforderlichen Einrichtungen, Vorrichtungen, Apparate oder anderen Gegenstände auf oder sind hiermit verbunden.

In bestimmten erfindungsgemäßen Ausführungsformen des Verfahrens wird das Aufbauen eines Drucks mittels der Hydraulikeinrichtung oder der Pneumatikeinheit innerhalb der Fluidkommunikation nur ausgeführt, wenn bei einem vorausgehenden Anlegen eines Vordrucks, welcher niedriger als der aufzubauende Druck ist, keine - oder gemessen an einer Vorgabe eine zu große - Funktionsabweichung festgestellt wurde. Eine zu große Funktionsabweichung liegt beispielsweise dann vor, wenn bereits bei Anlegen eines Vordrucks eine Leckage erkannt wird.

Das Verfahren umfasst in einigen erfindungsgemäßen Ausführungsformen das Befüllen, insbesondere das vollständige Befüllen, der Dialysatseite und/oder der Blutseite eines Blutfilters, mit welchem die Funktionseinrichtung in Fluidkommunikation verbunden ist.

In gewissen erfindungsgemäßen Ausführungsformen umfasst das Verfahren das Aufbauen des Drucks mittels einer Substituatpumpe der Behandlungsvorrichtung.

Das Verfahren umfasst in einigen erfindungsgemäßen Ausführungsformen das Aufbauen des Drucks gegen die stehende oder allenfalls definiert fördernde Substituatpumpe.

In manchen erfindungsgemäßen Ausführungsformen umfasst das Verfahren das Feststellen oder Messen eines Druckabfalls oder eines Druckanstiegs innerhalb der Fluidkommunikation. Es umfasst ferner, dass eine Durchgängigkeit (oder mangelnde Durchgängigkeit) der Fluidkommunikation oder der Funktionseinrichtung festgestellt oder postuliert wird aufgrund eines Vergleichs des festgestellten Druckabfalls oder Druckanstiegs mit zuvor gespeicherten Werten, Schwellenwerten, Bereichen oder Verläufen.

Das Verfahren umfasst in bestimmten erfindungsgemäßen Ausführungsformen das Feststellen oder Messen eines Druckabfalls oder Druckanstiegs - insbesondere innerhalb eines vorbestimmten Zeitraums - innerhalb der Fluidkommunikation. Es umfasst ferner das Feststellen (Feststellen ist hierin auch synonym zu einem Erkennen, einem Postulieren usw. verwendet oder zu verstehen) einer Dichtigkeit oder einer mangelnden Dichtigkeit der Fluidkommunikation oder der Funktionseinrichtung. Dieses Feststellen basiert auf einem Vergleich des festgestellten Druckabfalls oder Druckanstiegs mit zuvor gespeicherten Werten, Schwellenwerten, Bereichen oder Verläufen.

In gewissen erfindungsgemäßen Ausführungsformen erfolgt das Feststellen eines Druckabfalls oder Druckanstiegs innerhalb der Fluidkommunikation innerhalb eines vorbestimmten Zeitraums.

Das Verfahren umfasst in erfindungsgemäßen Ausführungsformen das Fördern eines vorbestimmten Fördervolumens innerhalb der Fluidkommunikation bei konnektiertem automatischem Substituatkonnektor bei oder mittels rückwärts laufender Substituatpumpe gegen ein Rückschlagventil, welches innerhalb eines Substituatkanals vorgesehen ist. Das Verfahren dieser Ausführungsformen umfasst ferner das Feststellen einer Druckveränderung innerhalb der Fluidkommunikation. Es umfasst zudem das Feststellen einer Rückschlagfunktion (oder Nichtfunktion) des Rückschlagventils für den Fall, dass ein festgestellter Druckabfall nicht gleich oder höher als ein maximaler Druckabfall ist. Alternativ kann zur Überprüfung der Rückschlagfunktion natürlich auch ein Druckanstieg hinter dem Rückschlagventil überwacht werden.

Unter einer rückwärts laufenden Pumpe, z. B. Substituatpumpe, ist eine Pumpe zu verstehen, welche in einer Richtung entgegengesetzt zu jener Richtung (vorwärts) fördert, in welcher die betrachtete Pumpe während der Behandlung des Patienten, jedenfalls ganz überwiegend, fördert.

In erfindungsgemäßen Ausführungsformen umfasst das Verfahren das Fördern eines vorbestimmten Fördervolumens bei konnektiertem automatischem Substituatkonnektor innerhalb der Fluidkommunikation bei rückwärts laufender Substituatpumpe gegen ein Rückschlagventil, welches innerhalb eines Substituatkanals vorgesehen ist. Es umfasst zudem das Feststellen einer Druckveränderung innerhalb der Fluidkommunikation sowie das Feststellen, dass die Substituatpumpe vorwärts fördert oder gefördert hat, falls ein festgestellter Druckabfall höher als ein maximaler Druckabfall ist.

Das erfindungsgemäße Verfahren umfasst in bestimmten Ausführungsformen das Ermitteln eines aktuellen Druckwertes eines späteren, zweiten Zeitpunkts ausgehend von einem bereits vorliegenden Druckwert eines früheren, ersten Zeitpunkts, von einem Messwert und von einem vorbestimmten Glättungsfaktor oder Multiplikator - insbesondere während einer Druckhaltephase - nach der Formel:

$$\text{WERT\_neu} = \text{WERT\_alt} * \text{GF} + \text{MESSWERT} * (1 - \text{GF})$$

wobei gilt:

WERT_neu ist ein aktueller Druckwert, er kann ein fiktiver oder errechneter Wert sein;

WERT_alt ist der bereits vorliegende oder zu einem früheren Zeitpunkt ermittelte Druckwert, er kann ebenfalls ein fiktiver oder - insbesondere mit der hier diskutierten Formel - errechneter Wert sein;

GF ist ein Glättungsfaktor; und

MESSWERT ist ein aktuell, also zum späteren, zweiten Zeitpunkt gemessener Druck.

In gewissen erfindungsgemäßen Ausführungsformen des Verfahrens liegt der Glättungsfaktor in einem Bereich von 0,9 bis 0,96, insbesondere entspricht er 0,93.

Das Verfahren umfasst in einigen erfindungsgemäßen Ausführungsformen das Befüllen eines mit der Funktionseinrichtung verbundenen extrakorporalen Blutkreislaufs - vorzugsweise so, dass dieser luftfrei ist - vor Aufbauen des Drucks.

In manchen erfindungsgemäßen Ausführungsformen des Verfahrens wird wenigstens eine Funktion, insbesondere das Öffnen, das Schließen oder eine Dichtfunktion, wenigstens des Ventils für eine Zugabestelle für Substituat in Prädilution; eines Ventils für eine Zugabestelle für Substituat in Postdilution; eines Single-Needle-Ventils; eines Rückschlagventils im Substituatkanal; und/oder eines automatischen Substituatkonnektors überprüft.

In bestimmten erfindungsgemäßen Ausführungsformen wird das Verfahren vor Aufnahme einer Behandlung eines Patienten, bei welcher die Funktionseinrichtung zum Einsatz kommt, durchgeführt.

Das Verfahren umfasst in erfindungsgemäßen Ausführungsformen das Sperren von Behandlungsmodalitäten und/oder das Einschränken von Behandlungsparametern des Behandlungsverfahrens oder der Behandlungsmodalität. Das Sperren oder Einschränken erfolgt basierend auf (oder unter Einbezug von) einem Ergebnis der Überprüfung wenigstens einer Funktion der medizinischen Funktionseinrichtung und/oder der Behandlungsvorrichtung durch das vorliegende Verfahren.

In erfindungsgemäßen Ausführungsformen des Verfahrens ist die medizinische Funktionseinrichtung eine Blutkassette und die Behandlungsvorrichtung eine Blutbehandlungsvorrichtung.

In manchen erfindungsgemäßen Ausführungsformen weist die Erfassungseinrichtung wenigstens eine Anzeigeeinrichtung zum Anzeigen eines Ergebnisses der Durchführung des erfindungsgemäßen Verfahrens auf. Die Anzeigeeinrichtung kann ein Display, eine Fehleranzeige oder dergleichen sein.

Die Erfassungseinrichtung weist in bestimmten erfindungsgemäßen Ausführungsformen wenigstens eine Alarmeinrichtung zum Ausgeben eines Alarms auf. Die Alarmeinrichtung kann vorgesehen oder konfiguriert sein zum Ausgeben eines Alarms für den Fall, dass das Ergebnis der Durchführung des erfindungsgemäßen Verfahrens nicht in einem vorbestimmten Bereich oder Wertebereich liegt. Der Alarm kann ein akustischer und/oder ein optischer Alarm sein.

In gewissen erfindungsgemäßen Ausführungsformen ist die Erfassungseinrichtung programmiert und/oder konfiguriert, um derart auf die Behandlungsvorrichtung einzuwirken, dass wenigstens eine Behandlungsoption oder -modalität (z. B. ein Dialyseverfahren, insbesondere wie hierin aufgezählt), zu welcher die überprüfte Funktionseinrichtung bestimmungsgemäß oder konstruktionsbedingt (auch) einsetzbar ist, und/oder das Behandeln unter vorbestimmten Behandlungsparametern mittels der konkreten, überprüften Funktionseinrichtung nicht durchführbar ist. Dies gilt für den Fall, dass eine mangelnde Funktionsfähigkeit - z. B. ein ungenügendes Schließen, Öffnen oder Dichten - der Funktionseinrichtung und/oder der Behandlungsvorrichtung (oder jeweils einer Komponente hiervon, insbesondere des Ventils der Zugabestelle für Substituat in Prädilution und/oder eines Ventils der Zugabestelle für Substituat in Postdilution) in diesem Verfahren erkannt wurde.

Mittels der erfindungsgemäßen Erfassungseinrichtung wird in einigen Ausführungsformen derart auf die Behandlungsvorrichtung eingewirkt, dass die Behandlungsoption(en) Hämofiltration und/oder Hämodiafiltration nicht mittels der konkreten, geprüften Funktionseinrichtung durchführbar sind.

In manchen erfindungsgemäßen Ausführungsformen ist die Erfassungseinrichtung eine Steuerungs- oder Regelungsvorrichtung und/oder ein Funktionstestmonitor oder weist jeweils eine(n) solche(n) auf.

In bestimmten erfindungsgemäßen Ausführungsformen ist die medizinische Behandlungsvorrichtung als Blutbehandlungsvorrichtung, insbesondere als Vorrichtung zur Apherese oder Dialyse, wiederum insbesondere zur Hämodialyse, Hämofiltration, Hämodiafiltration, ausgestaltet.

Einige oder alle erfindungsgemäßen Ausführungsformen können einen oder mehrere der oben oder im Folgenden genannten Vorteile aufweisen.

So kann, da erfindungsgemäß sicherheitsrelevante Funktionen der verwendeten Artikel sowie der Behandlungsvorrichtung geprüft werden, eine erhöhte Sicherheit für den Patienten geschaffen werden.

Da das erfindungsgemäße Verfahren beim Vorbereiten oder Aufrüsten der Behandlungsvorrichtung erfolgen kann, kann ein technischer Fehler bereits erkannt werden, bevor der Patient konnektiert ist und bevor Blut in Kontakt mit der Behandlungsvorrichtung sowie dem extrakorporalen Blutkreislauf gelangt ist. Letzteres vermeidet einen unnötigen Mehrverbrauch an Einwegartikeln. Es erlaubt ferner ein einfaches, da frühzeitiges Austauschen von defekten Komponenten im Fehlerfall.

Da das erfindungsgemäße Verfahren in erfindungsgemäßen Ausführungsformen ohne jedes Zutun des diensthabenden Personals erfolgen kann, sondern automatisch abläuft, ist es vorteilhaft möglich, Fehler arbeitszeitsparend und bereits vor Behandlungsbeginn zu erkennen. Zudem erlaubt das hierin beschriebene Vorgehen dadurch, dass es automatisiert ausführbar ist, dass relevante Testschritte oder -prozeduren nicht vergessen werden können. Die vorliegende Erfindung wird im Folgenden anhand der beigefügten Zeichnungen, in welchen identische Bezugszeichen gleiche oder ähnliche Bauteile bezeichnen, exemplarisch erläutert. In den zum Teil stark vereinfachten Figuren gilt:

Fig. 1     zeigt ein schematisch vereinfachtes Schaubild einer erfindungsgemäßen Behandlungsvorrichtung mit einer Blutkassette, welche mittels der vorliegenden Erfindung geprüft werden kann; und

Fig. 2     zeigt schematisch vereinfacht einen Pneumatik-Schaltplan einer erfindungsgemäßen Behandlungsvorrichtung.

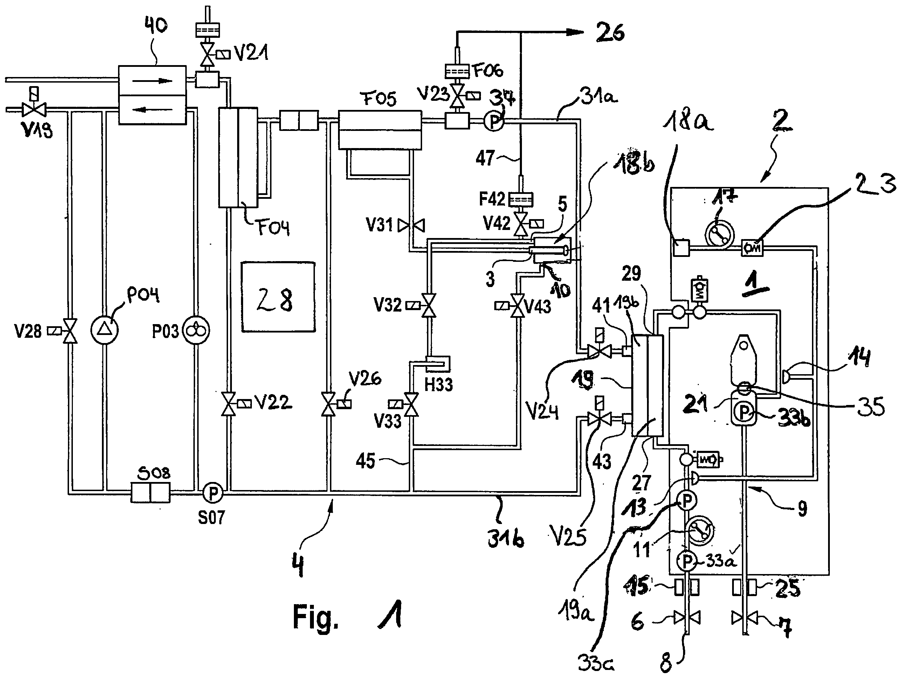

Fig. 1 zeigt ein Schaubild einer erfindungsgemäßen Behandlungsvorrichtung mit einer Blutkassette sowie deren Komponenten, welche zum besseren Verständnis der im Folgenden erläuterten exemplarischen Ausführungsformen der vorliegenden Erfindung erläutert werden.

Ein extrakorporaler Blutkreislauf 1 verläuft außerhalb und innerhalb einer Blutkassette 2 und verbindet diese mit einer Behandlungsvorrichtung 4. Der extrakorporale Blutkreislauf 1 weist eine Zugangseinrichtung (nicht gezeigt), beispielsweise eine arterielle Konnektionsnadel, auf oder ist hiermit verbunden. Ein Fluidstrom durch den extrakorporalen Blutkreislauf 1 oder Abschnitte hiervon kann mittels einer arteriellen Patientenschlauchklemme 6, angeordnet in dessen arteriellem Patientenschlauch 8 (auch als arterielle Blutleitung bezeichnet), unterbunden werden, ferner mittels einer venösen Patientenschlauchklemme 7 in dessen venösem Patientenschlauch 9 (auch als venöse Blutleitung bezeichnet).

Ein Abschnitt des extrakorporalen Blutkreislaufs 1 ist in eine Blutpumpe 11 eingelegt. Der extrakorporale Blutkreislauf 1 weist eine Zugabestelle 13 für Substituatflüssigkeit (in Prädilution) und eine Zugabestelle 14 für Substituatflüssigkeit (in Postdilution) auf. Die Zugabestellen 13 und 14 sind hier exemplarisch als Phantomventile ausgeführt. Phantomventile dieser Art sind in der WO 2010/121819 der Anmelderin auch der vorliegenden Erfindung beschrieben. Für Details wird auf jene Offenlegungsschrift verwiesen.

Ein arterieller Luft-Blut-Detektor 15 ist an der arteriellen Blutleitung 8 vorgesehen.

Fig. 1 zeigt ferner eine Substituatpumpe 17. Diese liegt stromabwärts einer Verbindungsstelle, an welcher die Blutkassette 2 vor deren Gebrauch mit ihrem Substituatport 18a mit einem automatischen Substituatkonnektor 18b der Behandlungsvorrichtung 4 verbunden werden kann. Der automatische Substituatkonnektor 18b weist im Beispiel der Fig. 1 eine erste Fluidführung 3, eine zweite Fluidführung 5 und eine dritte Fluidführung 10 zum Spülen des automatischen Substituatkonnektors 18b und zum Leiten von Substituat durch den automatischen Substituatkonnektor 18b hindurch auf.

In den extrakorporalen Blutkreislauf 1 ist ein Blutfilter 19 mit einer Blutkammer 19a und einer Dialysatkammer 19b eingeschaltet.

Die Blutkassette 2 weist eine venöse Luftabscheidekammer 21 auf.

Eine Substituatleitung der Blutkassette 2 weist ein Rückschlagventil 23 auf.

Der extrakorporale Blutkreislauf 1 weist einen venösen Luft-Substituatflüssigkeit-Blut-Detektor 25 an der venösen Blutleitung 9 auf.

Die Behandlungsvorrichtung 4 weist eine Druckluftquelle 26 auf oder ist hiermit verbunden. Sie weist ferner eine Steuerungs- oder Regelungseinrichtung 28 auf oder ist hiermit verbunden.

In Fig. 1 sind ferner eine Dialysierflüssigkeitszulaufleitung 31a, welche Dialysierflüssigkeit zur Dialysatkammer 19b führt, und eine Dialysatablaufleitung 31b, welche Dialysat von der Dialysatkammer 19b wegführt, zu erkennen.

Ein Drucksensor 33a ist stromaufwärts der Blutpumpe 11 in der arteriellen Blutleitung 8 vorgesehen.

Ein Drucksensor 33b ist im Bereich der venösen Luftabscheidekammer 21 in der venösen Blutleitung 9 vorgesehen.

Ein Drucksensor 33c, auch als Präfilterdrucksensor bezeichnet, ist stromabwärts der Blutpumpe 11 in der arteriellen Blutleitung 8 vorgesehen. Dieser kann stromaufwärts der Zugabestelle 13 angeordnet sein.

Die Blutkassette 2 weist ein Single-Needle-Ventil 35 auf.

Ein wiederum weiterer Drucksensor 37 befindet sich in oder an der Dialysierflüssigkeitszulaufleitung 31a zwischen der Druckquelle 26 und dem Blutfilter 19.

Ventile V19, V24, V25, V28, V31, V32 und V33 sind in Abschnitten der Hydraulik der Behandlungsvorrichtung 4 vorgesehen.

Das Ventil V24 liegt in oder an der Dialysierflüssigkeitszulaufleitung 31a.

Das Ventil V25 liegt in oder an der Dialysatablaufleitung 31b.

Das Ventil V28 liegt in einer Drain-Leitung 45.

Das Ventil V31 liegt in der ersten Fluidführung 3 des automatischen Substituatkonnektors 18b oder in einer zu diesem hin führenden Leitung.

Das Ventil V32 liegt in der zweiten Fluidführung 5 des automatischen Substituatkonnektors 18b oder in einer zu diesem hin führenden Leitung.

Das Ventil V33 liegt wiederum in der Drain-Leitung 45.

Das Ventil V19 liegt stromabwärts aller vorstehend genannten Ventile V24, V25, V28, V31, V32 und V33.

Nur angedeutet ist eine Hydraulik-Bilanzkammer 40.

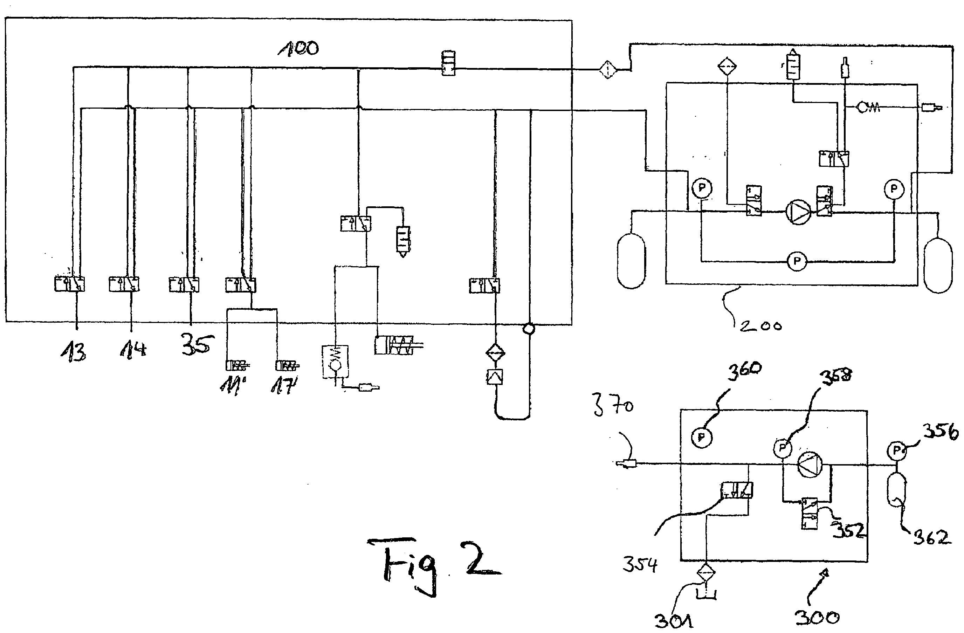

Fig. 2 zeigt einen Pneumatik-Schaltplan einer erfindungsgemäßen Behandlungsvorrichtung. Die hierin gezeigten Komponenten dienen wiederum dem besseren Verständnis der im Folgenden erläuterten exemplarischen Ausführungsformen der vorliegenden Erfindung.

In Fig. 2 dargestellt ist eine Luftverteilerplatte 100, welche mit einer Pneumatikeinheit 200 verbunden ist. Dargestellt ist ferner eine Single-Needle-Unit 300.

Die Luftverteilerplatte 100 weist Verbindungen zur Zugabestelle 13, zur Zugabestelle 14, zum Single-Needle-Ventil 35, zu einer Bremseinrichtung 11' der Blutpumpe 11, zu einer Bremseinrichtung 17' der Substituatpumpe 17, sowie zu weiteren, hier nicht weiter diskutierten Bauteilen auf.

Die Pneumatikeinheit 200 ist als Druckluftquelle 26 (siehe Fig. 1) mit der Hydraulik der Behandlungsvorrichtung 4 verbunden.

Die Single-Needle-Unit 300 weist eine Be- und Entlüftung 301, einen Single-Needle-Kompresser 350, ein Bypass-Ventil 352, ein Entlüftungsventil 354, einen Drucksensor 356 am Single-Needle-Druckbehälter, einen Drucksensor 358 der SN-Leitung, einen Absolutdrucksensor 360, sowie einen Temperatursensor 362 im SN-Druckbehälter auf. Ein Verbinder 370 verbindet die Single-Needle-Unit 300 mit der Single-Needle-Kammer der Blutkassette 2.

Zur Bedeutung der übrigen in den Figuren bezeichneten Elemente wird auf die unten stehende Bezugszeichenerklärung in Listenform verwiesen.

Das folgende erste Ausführungsbeispiel betrifft einen Dichtigkeitstest bei einem Disposable, hier einer Blutkassette 2, welches keine Single-Needle(SN)-Kammer aufweist oder bei welchem eine Single-Needle-Kammer durch ein Single-Needle-Ventil 35 vom extrakorporalen Blutkreislauf 1 abgetrennt ist und bleibt. Hierbei wird geprüft, ob die Blutkassette 2 nach außen dicht oder ausreichend dicht ist. Ferner wird bei geschlossener und verpresster Tür bei eingelegter Blutkassette 2 geprüft, ob ein Druck im extrakorporalen Blutkreislauf 1 (kurz auch: EB 1, wobei 1 das Bezugszeichen angibt) aufgebaut und gehalten werden kann. Mittels dieses Tests kann insbesondere geprüft werden, ob die Dichtigkeit des extrakorporalen Blutkreislaufs 1 (ohne Patientenschläuche) gegeben ist, die Stillstandsleckage beider Blutpumpenrollen, und/oder ob eine ausreichende Verpressung zur Dichtung der Flusswege und deren Verzweigungen in der Blutkassette 2 besteht.

Zudem kann mittels des unten beschriebenen Vorgehens auch die Funktion des Rückschlagventils 23 im Substituatkanal geprüft werden, insbesondere die Funktionen "Schließen" und "Dichten" des Rückschlagventils 23 in der Blutkassette 2.

Zur Durchführung des Tests wird die Blutkassette 2 in die Behandlungsvorrichtung eingelegt und durch Schließen einer Tür der Behandlungsvorrichtung gegen diese verpresst. Der Dialysator oder Blutfilter 19 ist disposable- und hydraulikseitig konnektiert und gefüllt. Der extrakorporale Blutkreislauf 1 ist mit Flüssigkeit gefüllt und weitgehend luftfrei. Der EB 1 ist alarmfrei, das Blutsystem ist aktiv, die Hydraulik befindet sich in einem Stadium der Vorbereitung, der Dialysatfluss ist eingeschaltet. Der arterielle Patientenschlauch 8 und der venöse Patientenschlauch 9 sind beide über ein T-Stück mit dem nicht dargestellten Spülport verbunden.

Zu Beginn des Tests dieser erfindungsgemäßen Ausführungsform befindet sich der automatische Substituatkonnektor 18b in Konnektionsstellung. Die Substituatzufuhrleitungen oder der Substituatzweig ist bzw. sind gefüllt.

Das Ventil V33 ist geschlossen. Die Hydraulik wird in den sog. Extended Bypass versetzt (die Ventile V24, V25 sind stromlos). Das Ventil V31 ist offen. Die arterielle Patientenschlauchklemme 6 und die venöse Patientenschlauchklemme 7 sind offen (bestromt). Das Prädilutionsventil 13 und das Postdilutionsventil 14 sind offen, das Single-Needle-Ventil 35 ist geschlossen.

Übersteigt der Druck in der Hydraulik den vorbestimmten Druck PTESTSTART_HYD (gemessen mittels Drucksensor 37, angeordnet an der Dialysierflüssigkeitszulaufleitung 31a), so wird dieser mithilfe der Substituatpumpe 17 abgebaut. Dazu wird V33 geöffnet und so lange Substituat gefördert, bis der Hydraulikdruck (wiederum gemessen mittels Drucksensor 37) unter den Druck PTESTSTART_HYD gefallen ist. Anschließend wird die Substituatpumpe 17 gestoppt und V33 wieder geschlossen.

Übersteigt der Druck in der Hydraulik den vorbestimmten Druck PTESTSTART_HYD nicht, so wird geprüft, ob der Druck im EB 1 den vorbestimmten Druck PTESTSTART_EB (gemessen am Drucksensor 33b im Bereich der venösen Luftabscheidekammer 21) übersteigt. Ist dies der Fall, so wird dieser über das Öffnen des Ventils V33 der Hydraulik abgebaut; anschließend wird das Ventil V33 geschlossen.

Daraufhin wird der Blutpumpenrotor der Blutpumpe 11 so positioniert, dass nur eine erste Rolle des Pumpenrotors im Eingriff mit dem extrakorporalen Blutkreislauf 1 ist. Dann werden sowohl die arterielle Patientenschlauchklemme 6 als auch die venöse Patientenschlauchklemme 7 geschlossen und zum Druckausgleich eine vorbestimmte Wartezeit DRUCKAUSGLEICHZEIT abgewartet, und es wird zur Ermittlung der Rollenstillstandsleckage der arterielle Startdruck (gemessen mittels Drucksensor 33a der arteriellen Blutleitung) notiert.

Mit der Substituatpumpe 17 wird mittels eines vorbestimmten Flusses SUBSTITUATPUMPE: FLUSS Flüssigkeit in den EB 1 gefördert. Zunächst wird mittels des Präfilterdrucksensors 33c geprüft, ob innerhalb eines definierten Zeitintervalls TIMEOUT ein Prüfdruck PRUEFDRUCK im EB 1 aufgebaut werden kann.

Ist dies der Fall, wird nach diesem bestandenen Zwischentest die Substituatpumpe 17 angehalten. Danach wird eine angemessene Zeit DRUCKAUSGLEICHZEIT abgewartet, damit sich ein statischer Druck ausbilden kann. Ist während der Druckausgleichsphase der Druck unter den minimalen Prüfdruck MIN PRUEFDRUCK abgefallen, so wird der Test mit einem Fehler abgebrochen.

Andernfalls wird der nun folgende Testschritt für die sich von der ersten Rolle unterscheidende zweite Rolle des Pumprotors durchgeführt. Es wird der Druckabfall innerhalb einer definierten Zeitspanne TESTTIME ermittelt. Betrachtet wird die absolute Druckänderung, wobei diese einen maximalen Wert MAXIMALER DRUCKABFALL nicht überschreiten darf.

Demgegenüber wird beim Prüfen der ersten Rolle eine Zeitdauer TESTTIME abgewartet, ohne dass der Druckabfall bewertet wird.

Nach bestandener Prüfung wird der arterielle Druckanstieg bewertet. Überschreitet dieser den vorgegebenen Druckabfall MAXIMALE STILLSTANDSLECKAGE, so liegt keine vollständige Okklusion vor.

Der folgende Testschritt wird nur für die erste Rolle des Pumprotors durchgeführt. Dabei wird getestet, ob das Rückschlagventil 23 im Substituatkanal schließt und dichtet.

Dabei bleibt der vorher aufgebaute Prüfdruck erhalten. Mit der Substituatpumpe 17 wird rückwärts gefördert mit dem Ziel, ein maximales Fördervolumen MAX FOERDERVOLUMEN zu fördern, und gleichzeitig wird der absolute Druckabfall, gemessen am Drucksensor 33c, bewertet. Dieser darf einen Höchstwert MAXIMALE DRUCKAENDERUNG oder dessen Betrag nicht überschreiten. Gleichzeitig darf der Druck in der Blutkassette 2 nicht ansteigen (ist dies der Fall, so dreht die Substituatpumpe 17 verkehrt herum).

Da das Kompartiment zwischen Rückschlagventil 23 und benachbarter Substituatpumpe 17 sehr klein ist (< 3ml), ist das von der Substituatpumpe 17 förderbare Volumen im Regelfall begrenzt. Bei nicht schließendem Rückschlagventil 23 hängt das geförderte Volumen von der maximal zulässigen Druckänderung MAXIMALE DRUCKAENDERUNG ab. Für einen bestimmten Typ eines extrakorporalen Blutkreislaufs 1 (im luftfreien System) gilt, dass ein Druckabfall von 50 mbar in etwa einer Leckage von 250 µl entspricht.

Um den Druck im EB 1 abzubauen, werden die arterielle Patientenschlauchklemme 6, die venöse Patientenschlauchklemme 7 und das Ventil V33 bestromt.

Die gesamte Prüfung wird für die andere Rolle des Blutpumpenrotors wiederholt. Dazu wird zunächst der Druckabbau im EB 1 abgewartet (DRUCKAUSGLEICHZEIT), anschließend der Spülport geschlossen und mit dem Schritt zur Positionierung des Blutpumpenrotors fortgefahren.

Im Fehlerfall wird die Substituatpumpe 17 angehalten und der Druck im EB 1 durch das Öffnen der venösen Patientenschlauchklemme 7 und das Bestromen der Hydraulikventile V33, V28 und V19 abgebaut.

Versuche der Anmelderin haben ergeben, dass mittels dieses Tests eine Leckage im Bereich von 5,5 ml/min nach außen oder in die SN-Kammer hinein erkannt werden kann. Ferner kann es möglich sein, eine Nicht- und Halbverpressung der Blutkassette 2 zu erkennen.

Bei nicht bestandenem Test ist in einigen erfindungsgemäßen Ausführungsformen eine einmalige Wiederholung vorgesehen. Aus Sicherheitsgründen kann vorgesehen sein, dass eine Behandlung des Patienten erst bei bestandenem Test möglich ist.

Bei nicht bestandenem Test kann es erforderlich sein, die getestete Blutkassette 2 abzurüsten und gegen eine neue Blutkassette 2 auszutauschen. Die neue Blutkassette 2 wird erneut befüllt und gespült.

Beispiele für voreingestellte Werte der o. g. Parameter sind in der folgenden Parametertabelle (Tabelle 1) wiedergegeben:

Tabelle 1

| Untertest | Parameter | Einheit | Wert |
|---|---|---|---|
| allgemeine Parameter | DRUCKAUSGLEICHZEIT | ms | 2000 |
| | SUBSTITUATPUMPE: FLUSS | ml min$^{-1}$ | 500 |
| | PTESTSTART_EB | mbar | 67 |
| | PTESTSTART_HYD | mbar | 333 |
| "Druck aufbauen" | PRUEFDRUCK | mbar | 1400 |
| | MIN PRUEFDRUCK | mbar | 1250 |
| | TIMEOUT | ms | 15000 |
| "Dichtigkeitsprüfung" | TESTTIME | ms | 5000 |
| | MAXIMALER DRUCKABFALL | mbar | 73 |
| | MAXIMALE STILLSTANDSLECKAGE | mbar | 400 |
| "Prüfung Rückschlagventil 23" | MAX FOERDERVOLUMEN | ml | 5 |
| | MAXIMALE DRUCKAENDERUNG | mbar | 20 |

Beim vorliegenden Test auftretende Fehler und Vorschläge zu ihrer Behebung sind in der folgenden Fehlertabelle (Tabelle 2) wiedergegeben:

Tabelle 2

| Untertest | Fehler |
|---|---|
| "Druck aufbauen" | TIMEOUT überschritten<br>→ SN-Ventil 35 offen<br>→ Disposable nicht vollständig verpresst |
| "Dichtigkeitsprüfung" | MAXIMALER DRUCKABFALL überschritten<br>→ Disposable undicht |
| | MAXIMALE STILLSTANDLECKAGE überschritten<br>→ Rolle unvollständig im Eingriff |
| "Prüfung Rückschlagventil 23" | MAXIMALE DRUCKAENDERUNG überschritten<br>→ Rückschlagventil 23 nicht dicht oder<br>→ Substituatpumpe 17 dreht vorwärts |

Mit dem Test dieser Ausführungsform kann eine z. B. aufgrund nicht ausreichender Verpressung undichte Blutkassette 2 erkannt werden.

Zum Erkennen weiterer oder anderer Leckagen kann überdies ein Leckagensensor vorgesehen sein.

Die **zweite** erfindungsgemäße Ausführungsform betrifft einen Test zum Überprüfen der Dichtigkeit der Substituatkonnektion. In diesem Test wird geprüft, ob die Substituatkonnektion (automatischer Substituatkonnektor 18b (ASK) mit Substituatport 18a) nach außen hin dicht ist. Außerdem wird der Verschluss des Disposables auf Durchgang geprüft.

Hierzu ist die Blutkassette 2 in die Behandlungsvorrichtung eingelegt und verpresst. Die Rollen des Substituatrotors sind ausgefahren. Die Hydraulik ist verfügbar. Der automatische Substituatkonnektor 18b konnektiert. Mittels der Hydraulik wird nach der Konnektion der Blutkassette 2 ein Druckhaltetest durchgeführt.

Mittels dieses Tests ist es möglich, eine Leckage im Bereich des automatischen Substituatkonnektors 18b nach außen im Bereich von 1 ml/min (entspricht etwa 500 ml/Behandlung) zu erkennen. Zudem ist es möglich, einen Verschluss eines Online-Anschlusses in der Blutkassette 2 festzustellen.

Eine Behandlung des Patienten ist in einigen erfindungsgemäßen Ausführungsformen ohne bestandenen Test nicht möglich. Dieser kann beliebig oft wiederholt werden. Alternativ wird die Blutkassette 2 abgerüstet und gegen eine andere Blutkassette ausgetauscht.

Mit dem druckgesteuerten Füllprogramm der Bilanzkammer wird bei angekoppeltem Substituatkonnektor und offenem Ventil V31 Druck im Bereich des Drucksensors 37 gegen die stehende Substituatpumpe 17 aufgebaut. Dieser Druck darf während einer Haltephase nur innerhalb bestimmter Grenzen absinken. Um im Falle einer grob undichten Konnektion den Flüssigkeitsaustritt gering zu halten, wird in bestimmten erfindungsgemäßen Ausführungsformen ein Vortest mit geringem Druck vorangestellt.

Außerdem wird die Durchgängigkeit zwischen der Hydraulik der Behandlungsvorrichtung 4 und der Substituatpumpe 17 getestet, indem durch Verfahren oder Betreiben der Substituatpumpe 17 ein Druckabfall am Drucksensor 37 erwartet wird.

Zu oder vor Beginn des Tests sind das Ventil V31 und das Ventil V24 geschlossen.

Die Zugabestelle 13 ist ebenso wie die Zugabestelle 14 geschlossen. Die Substituatpumpe 17 steht.

Zum Starten des Tests wird ein druckgesteuertes Füllprogramm gestartet. Dabei wird ein Druckwert NIEDRIGE DRUCKGRENZEN angefordert. Es wird ein Zeitraum TIMEOUT von z. B. 2 s abgewartet, in welchem sich im Rahmen eines Schnelltests ein minimaler Druck NIEDRIGE DRUCKGRENZEN aufgebaut oder eingehalten haben soll. Sollte dies nicht der Fall sein, auch nicht innerhalb der Wartezeit TIMEOUT, so ist von einem Problem der Hydraulik auszugehen.

Der mittels Drucksensor 37 gemessene Druck kann gespeichert werden.

Anschließend wird das Ventil V31 mehrfach, z. B. dreimal (NV31), für kurze Zeit geschlossen und nach einer Wartezeit TV31 erneut geöffnet. Währenddessen muss ein vorbestimmter minimaler Druck MINDRUCK eingehalten werden. Anschließend bleibt V31 geöffnet, um zu sehen, ob ein vorbestimmter Druckabfall eingehalten wird. Wird der vorbestimmte maximale Druckabfall MAXDRUCKABFALL überschritten, oder wird ein vorbestimmter minimaler Druck MINDRUCK nicht eingehalten, so ist von einem Fehler auszugehen; der Test ist dann nicht bestanden, ggf. weil die Verbindung zum Substituatport 18a undicht ist. In diesem Testabschnitt kann der minimale Hydraulikdruck überwacht werden, um einen Druckaufbau durch das Füllprogramm auszuschließen.

Nach diesem Schnelltest wird für einen Durchlässigkeitstest zum Druckaufbau ein druckgesteuertes Füllprogramm mit einem Druck HOHE DRUCKGRENZEN gestartet. Wenn ein vorbestimmter Druck PRUEFDRUCK_HIGH am Drucksensor 37 gemessen wird, so wird weitergeschaltet. Wird der Mindestdruck am Sensor 37 nicht innerhalb einer Wartezeit PRUEFDRUCK-TIMEOUT aufgebaut, obwohl die Hydraulik verfügbar ist, so ist wiederum von einem Fehler auszugehen, beispielsweise von einem grob undichten Substituatport 18a.

Wird der erwartete Mindestdruck hingegen aufgebaut, so wird ein druckgesteuertes Füllprogramm mit niedrigen Druckgrenzen NIEDRIGE DRUCKGRENZEN angefordert. Hierbei muss nach Abwarten einer Wartezeit DRUCKAUSGLEICHZEIT geprüft werden, ob der mittels Drucksensor 37 gemessene Druck den erwarteten Mindestdruck MINPRUEFDRUCK unterschreitet. Ist dies der Fall, so ist von einem Fehler auszugehen, welcher ggf. von einer undichten Verbindung zum Substituatport 18a herrührt.

Nach Ablauf einer Totzeit DRUCKAUSGLEICHZEIT wird fortgefahren.

Der mittels Drucksensor 37 gemessene Druck kann gespeichert werden.

Zum Benetzen der Substituatpumpe und zum Testen der Blutkassette 2 auf ihre Durchlässigkeit wird in einem nächsten Schritt das Ventil für die Zugabestelle 14 geöffnet. Das Ventil für die Zugabestelle 13 bleibt geschlossen. Die Substituatpumpe 17 wird um ein vorgegebenes Volumen SPVOLUMEN vorwärts bewegt. Dazu kann ein geringer Fluss SUBSTITUATPUMPE: FLUSS eingestellt sein.

Bleibt der daraufhin mittels Drucksensor 37 festgestellte Druckabfall unter einem erwarteten Druckabfall oder unter einem Mindestdruckabfall MINDRUCKABFALL, so wird auf einen Fehler geschlossen; ggf. ist die Verbindung zum Substituatport 18a nicht durchgängig.

Andernfalls schreitet das Verfahren fort zu einer Dichtigkeitsprüfung. Hierzu wird das Ventil der Zugabestelle 14 geschlossen.

Es kann ein druckgesteuertes Füllprogramm mit einem Druck HOHE DRUCKGRENZEN geschaltet werden, welches wiederum der Überprüfung der Verbindung zum Substituatport 18a dient. Nach Ablauf der Wartezeit TIMEOUT, während welcher ein vorbestimmter Druck PRUEFDRUCK_HIGH aufgebaut werden muss, andernfalls wird von einer undichten Verbindung zum Substituatport 18a ausgegangen, wird weitergeschaltet zu einem wie oben beschriebenen Druckaufbau mittels eines druckgesteuerten Füllprogramms mit niedrigen Druckgrenzen. Wenn nach Ablauf einer Haltezeit MESSINTERVALL bestimmte Kriterien wie Druckabfall pro Sekunde unterhalb eines vorbestimmten Grenzwertes MAXLECKAGE erfüllt sind, gilt der Test als bestanden. Andernfalls, oder falls eine Wartezeit MAXHALTEZEIT überschritten wird, wird von einem Fehler ausgegangen; ggf. ist die Verbindung zum Substituatport 18a undicht.

Zur Druckwertermittlung während der Druckhaltephase kann wie folgt vorgegangen werden:

Der Druckwert wird geglättet. Dabei kommt eine Tiefpassfilterung mit dem Glättungsfaktor GF = 0,93 zum Einsatz:

$$WERT\_neu = WERT\_alt * GF + MESSWERT * (1 - GF)$$

Dieser Druckwert wird in jedem Slice aktualisiert; die Prüfung auf Unterschreitung der maximalen Leckagerate findet alle 2 Sekunden statt.

Beispiele für voreingestellte Werte der o. g. Parameter sind in der folgenden Parametertabelle (Tabelle 3) wiedergegeben:

Tabelle 3

| Untertest | Parameter | Einheit | Wert |
|---|---|---|---|
| allgemeine Parameter | PTESTSTART_HYD | mbar | 200 |
| | DRUCKAUSGLEICHZEIT | ms | 2000 |
| | NIEDRIGE DRUCKGRENZE | mbar | 250 |
| | PRUEFDRUCK_HIGH | mbar | 1100 |
| "Schnelltest" | TSCHALTZEIT | ms | 300 |
| | TSCHNELLTEST | ms | 1750 |
| | MAXDRUCKABFALL | % | 75 |
| | MINDRUCK | mbar | 67 |
| | TV31 | ms | 250 |
| | NV31 | Anzahl | 3 |
| "Test der Durchlässigkeit" | MINPRUEFDRUCK | mbar | 1050 |
| | TIMEOUT | ms | 2000 |
| | SUBSTITUATPUMPE: FLUSS | ml min$^{-1}$ | 250 |
| | SPVOLUMEN | ml | 12 |
| | MINDRUCKABFALL | mbar | 133 |
| "Dichtigkeitsprüfung" | PRUEFDRUCK-TIMEOUT | ms | 2000 |
| | MAXLECKAGERATE | mbar/s$^{-1}$ | 2,67 |
| | MESSINTERVALL | s | 2 |
| | MAXHALTEZEIT | s | 25 |

Beim vorliegenden Test auftretende Fehler und Vorschläge zu ihrer Behebung sind in der folgenden Fehlertabelle (Tabelle 4) wiedergegeben:

Tabelle 4

| Untertest | Fehler |
|---|---|
| "Schnelltest" | TIMEOUT überschritten<br>→ Prüfdruck konnte nicht aufgebaut werden<br>→ Hydraulikproblem |
| | MAXDRUCKABFALL überschritten oder MINDRUCK unterschritten<br>→ Druckabfall zu groß: Verbindung zum ASK grob undicht |
| "Test der Durchlässigkeit" | TIMEOUT überschritten<br>→ Verbindung zum ASK grob undicht<br>→ oder Hydraulikproblem |
| | MINPRUEFDRUCK unterschritten<br>→ Verbindung zum ASK grob undicht |
| | MINDRUCKABFALL nicht erreicht<br>→ Verbindung zum ASK nicht durchgängig |
| "Dichtigkeitsprüfung" | TIMEOUT überschritten<br>→ Verbindung zum ASK grob undicht<br>→ oder Hydraulikproblem |
| | während der HALTEZEIT wurde die MAXLECKAGE nicht unterschritten<br>→ Verbindung ASK-Disposable undicht |

Die **dritte** Ausführungsform betrifft einen Dichtigkeits- und Funktionstest der als Phantomventile ausgestalteten Zugabestellen 13 und 14 für Substituat in Prädilution bzw. in Postdilution. Es wird geprüft, ob die Phantomventile öffnen, schließen und dichten, bzw. ob Druckgradienten beim Öffnen bzw. Schließen der Phantomventile aufgebaut und gehalten werden können. Evtl. "hängende" bzw. nicht korrekt schließende Phantomventile können hierbei erkannt werden. Dieser Test ist beliebig oft wiederholbar. Wird er nachhaltig nicht bestanden, so kann abgerüstet und ein neues Disposable eingelegt werden. Alternativ kann ein Servicemitarbeiter informiert werden. Ohne bestandenen Test ist in bestimmten erfindungsgemäßen Ausführungsformen eine Behandlung des Patienten nicht möglich. Dieser Test kann auch dann durchgeführt werden, wenn die Ventile oder Zugabestellen nicht als Phantomventile ausgestaltet, sondern von einem anderen Ventiltyp sind.

Zum Durchführen dieses Tests wird die Blutkassette 2 eingelegt und wie oben beschrieben verpresst. Der Blutfilter 19 ist disposable- und hydraulikseitig konnektiert und gefüllt. Der extrakorporale Blutkreislauf 1 ist gefüllt und weitgehend luftfrei. Der extrakorporale Blutkreislauf 1 hat den Dichtigkeitstest der ersten Ausführungsform vorzugsweise bereits bestanden.

Der arterielle Patientenschlauch 8 und der venöse Patientenschlauch 9 sind jeweils über ein T-Stück mit dem Spülport verbunden.

Zu Beginn der Prüfung wird die Hydraulik in den Extended Bypass versetzt. Gleichzeitig werden die Ventile V31 und V33 bestromt und das druckgesteuerte Füllprogramm gestartet. Das Ventil V32 ist geschlossen.

Das Ventil der Zugabestelle 13 (Prädilution) wird geöffnet, das der Zugabestelle 14 (Postdilution) sowie das Single-Needle-Ventil 35 bleiben geschlossen. Geschlossen sind oder bleiben auch die arterielle Patientenschlauchklemme 6 und die venöse Patientenschlauchklemme 7.

Übersteigt der Druck in der Hydraulik einen vorbestimmten Druck PTESTSTART_HYD (gemessen mittels Drucksensor 37), so wird dieser mithilfe der Substituatpumpe 17 und einem Fluss SUBSTITUATPUMPE: FLUSS abgebaut. Die arterielle Patientenschlauchklemme 6 und die venöse Patientenschlauchklemme 7 können hierfür geöffnet werden. Jedenfalls wird das Ventil V33 geöffnet und solange Substituat gefördert, bis der Hydraulikdruck (gemessen mittels Drucksensor 37) unter den Druck PTESTSTART_HYD gefallen ist. Anschließend wird die Substituatpumpe 17 gestoppt und das Ventil V33 wieder geschlossen.

Übersteigt der Druck in der Hydraulik den vorbestimmten Druck PTESTSTART_HYD (gemessen mittels Drucksensor 37) nicht, so wird mittels Drucksensor 33b überprüft, ob der im extrakorporalen Blutkreislauf 1 gemessene Druck den Druck PTESTSTART_EB (gemessen am venösen Drucksensor 33b) übersteigt. Ist dies der Fall, so wird dieser über das Öffnen des Ventils V33 der Hydraulik abgebaut; anschließend wird das Ventil V33 geschlossen.

Zur Testung wird nun mit der Substituatpumpe 17 (SUBSTITUATPUMPE: FLUSS) der Zieldruck PRUEFDRUCK im extrakorporalen Blutkreislauf 1 aufgebaut, gemessen mittels des Drucksensors 33b. Dieser soll innerhalb der Wartezeit DRUCKAUFBAU-TIMEOUT erreicht sein. Wird am Drucksensor 33b kein solcher Druckaufbau festgestellt, so wird angenommen, dass das Ventil der Zugabestelle 13 nicht öffnet oder dass das Single-Needle-Ventil 35 offen steht.

Im Nichtfehlerfall wird die Zugabestelle 13 geschlossen, wobei die Substituatpumpe 17 weiter läuft. Im Normalfall ändert sich der Druck im extrakorporalen Blutkreislauf 1 nach Abwarten einer Wartezeit DRUCKAUSGLEICHZEIT während der sich anschließenden Wartezeit MESSDAUER nicht. Steigt er dennoch um einen Wert größer als der Druckanstieg MAXIMALER DRUCKANSTIEG (gemessen mittels Drucksensor 33b), so kann auf ein Problem mit dem/den Ventil(en) der Zugabestelle(n) 13 und/oder 14 geschlossen werden.

Ist die Zwischenprüfung bestanden, so wird die Substituatpumpe 17 angehalten und der Druck im extrakorporalen Blutkreislauf 1 durch das Öffnen der venösen Patientenklemme 7 und der Zugabestelle 13 abgebaut und die Wartezeit DRUCKAUSGLEICHZEIT abgewartet.

Die Prüfung der Zugabestelle 14 (Postdilution) erfolgt analog zu jener der Zugabestelle 13 (Prädilution).

Durch das Öffnen der venösen Patientenschlauchklemme 7 und durch das Anhalten der Substituatpumpe 17 wird der Druck im extrakorporalen Blutkreislauf 1 letztmalig abgebaut.

Im Fehlerfall wird durch das Öffnen der venösen Patientenschlauchklemme 7 und das Anhalten der Substituatpumpe 17 der Druck im extrakorporalen Blutkreislauf 1 abgebaut. Eine Hämofiltration (HF) und eine Hämodiafiltration (HDF) sind dann nicht mehr möglich.

Beispiele für voreingestellte Werte der o. g. Parameter sind in der folgenden Parametertabelle (Tabelle 5) wiedergegeben:

Tabelle 5

| Untertest | Parameter | Einheit | Wert |
|---|---|---|---|
| Allgemeine Parameter | DRUCKAUSGLEICHZEIT | ms | 2000 |
| | SUBSTITUATPUMPE: FLUSS | ml min$^{-1}$ | 100 |
| | PTESTSTART_EB | mbar | 67 |
| | PTESTSTART_HYD | mbar | 333 |
| "Öffnet Ventil 13?" | PRUEFDRUCK | mbar | 300 |
| | DRUCKAUFBAU-TIMEOUT | ms | 10000 |
| "Schließt Ventil 13? Dichten Ventil 13 & Ventil 14?" | MAXIMALER DRUCKANSTIEG | mbar | 4 |
| | MESSDAUER | ms | 5000 |
| "Öffnet Ventil 14?" | DRUCKAUFBAU-TIMEOUT | ms | 5000 |
| | PRUEFDRUCK | mbar | 300 |

Beim vorliegenden Test auftretende Fehler und Vorschläge zu ihrer Behebung sind in der folgenden Fehlertabelle (Tabelle 6) wiedergegeben:

Tabelle 6

| Untertest | Fehler |
|---|---|
| "Öffnet Ventil 13?" | DRUCKAUFBAU-TIMEOUT überschritten<br>→ Ventil 13 nicht offen |
| "Schließt Ventil 13? Dichten Ventil 13 & Ventil 14?" | Druck gestiegen um MAXIMALER DRUCKANSTIEG<br>→ Ventil 13 offen<br>→ Ventil 14 offen<br>→ Ventil 13 und/oder Ventil 14 undicht |
| "Öffnet Ventil 14?" | PRUEFDRUCK nicht erreicht<br>→ Ventil 14 nicht offen |

Die **vierte** Ausführungsform betrifft einen Dichtigkeitstest für das Single-Needle(SN)-System. Geprüft wird mittels Testluft, ob der SN-Bereich nach außen dicht ist.

Für diesen Test ist die Blutkassette 2 eingelegt und wie oben beschrieben verpresst. Das Single-Needle-Ventil 35 ist geschlossen. Es wird geprüft, ob bei verpresster Blutkassette 2 ein Druck im Single-Needle-Über- und Unterdruckkompartiment aufgebaut und gehalten werden kann. Lecks des SN-Systems von 500 ml/4 h und mehr können erkannt werden.

Bei nicht bestandenem Test kann dieser beliebig oft wiederholt werden. Alternativ kann der SN-Betrieb unterbunden oder verhindert werden. Wie stets kann der Test bei Nichtbestehen mit neuem Disposable wiederholt werden oder der Service informiert werden.

Es ist in bestimmten erfindungsgemäßen Ausführungsformen möglich, diesen Test mit dem Funktionstest "SN-Pneumatiksystem" der sechsten Ausführungsform zu kombinieren.

Die Flüssigkeitsmenge oder ihr Volumen in der Single-Needle-Kammer beträgt maximal 10 ml.

Zu Beginn des Tests wird das Bypass-Ventil 352 stromlos geschaltet (= geöffnet) und das Entlüftungsventil 354 bestromt (= geöffnet). Das Single-Needle-Ventil 35 wird geschlossen. Das Single-Needle-System wird somit inklusive SN-Tank auf Umgebungsdruck gebracht. Dies ist dann der Fall, wenn die SN-Drucksensoren 358 und 356 maximal um einen Wert OFFSET_MAX von Null abweichen. Dieser Vorgang muss nach einer Wartezeit TIMEOUT_INIT abgeschlossen sein. Andernfalls könnten die Ausgangsbedingungen nicht hergestellt werden, beispielsweise weil Entlüftungsventil 354 und/oder das Bypass-Ventil 352 nicht korrekt öffnen.

Für die Dichtigkeitsprüfung werden zum Aufbau eines Prüfdrucks das Entlüftungsventil 354 und das Bypass-Ventil 352 geschlossen. Anschließend wird eine Wartezeit DRUCKAUSGLEICHZEIT_KURZ abgewartet und danach die Startmasse (Gesamtluftmasse) im Single-Needle-System mit folgender Formel errechnet (Gleichung für ideale Gase, isotherm):

$$m = \frac{pV}{R_S T} \qquad\qquad m[\mu g] = \frac{p[Pa] \cdot V[mm^3]}{R_S \left[\dfrac{J}{kg \cdot K}\right] \cdot T[K]}$$

$$M_{ges} = m_{over} + m_{under} = \frac{p_{over} \cdot V_{over} + p_{under} \cdot V_{under}}{T \cdot R_S}$$

$R_s$ = spez. Gaskonstante für trockene Luft = $287\dfrac{J}{kg \cdot K}$

$P_{over}$ = Druck im Überdruckkompartiment (abs.) = gemessen mit Drucksensor 358

$P_{under}$ = Druck im Unterdruckkompartiment (abs.) = gemessen mit Drucksensor 356

$V_{over}$ = Volumen des Überdruckkompartiments = 87 ml

$V_{under}$ = Volumen des Unterdruckkompartiments = 308 ml

T = Umgebungstemperatur (K) = gemessen mit Temperatursensor 362

Der SN-Kompressor 350 wird mit einer Spannung SN-KOMPRESSOR: SPANNUNG, was gleichbedeutend ist mit einer Kompressorrate SN-KOMPRESSOR: RATE, gestartet. Im geschlossenen System wird hierdurch innerhalb einer Wartezeit TIMEOUT_DP ein Druck (gemessen mittels des Drucksensors 358) gleich oder oberhalb eines Prüfdrucks PRUEFDRUCK aufgebaut. Ist dieser Druck erfolgreich aufgebaut, so wird der SN-Kompressor 350 gestoppt, die Wartezeit DRUCKAUSGLEICHZEIT_LANG abgewartet. Im Anschluss daran wird die Luftmasse (M_ges(tStart)) im SN-System nach dem Druckaufbau berechnet und mit der Startmasse verglichen. Die Massedifferenz M_ges_0 - M_ges(tStart) darf dabei einen Wert DM_BUILD_FALL nicht unterschreiten und einen Wert DM_BUILD_RISE nicht übersteigen. Andernfalls ist die Massenänderung im System während des Druckaufbaus zu ausgeprägt.

Zur Leckagemessung wird nach Ablauf einer Wartezeit MESSDAUER die Luftmasse im System (M_ges_(tEnd)) erneut berechnet und mit der Startmasse zu Beginn des Druckhaltetests (M_ges(tStart)) verglichen. Der Betrag der Massedifferenz M_ges (tStart) - M_ges (tEnd) darf dabei einen Wert DM_TEST_MAX nicht überschreiten. Übersteigt er diesen Wert, so ist die Masseänderung im System während des Druckhaltetests zu hoch.

Eine Bewertung des "Masseverlustes" und keine direkte Bewertung der Druckänderung der jeweiligen Kompartimente kann aufgrund einer zulässigen (weil nicht auszuschließenden) leichten Leckage des SN-Kompressors vom Überdruck- in das Unterdruckkompartiment erfolgen.

Zum Abschluss des Tests wird das Bypass-Ventil 352 stromlos geschaltet, und das Single-Needle-System durch das Bestromen des Entlüftungsventils 354 belüftet.

Beispiele für voreingestellte Werte der o. g. Parameter sind in der folgenden Parametertabelle (Tabelle 7) wiedergegeben:

Tabelle 7

| Untertest | Parameter | Einheit | Wert |
|---|---|---|---|
| allgemeine Parameter | DRUCKAUSGLEICHZEIT_LANG | s | 5 |
| | DRUCKAUSGLEICHZEIT_KURZ | s | 1 |
| "Ausgangsbedingungen herstellen" | OFFSET_MAX | mbar | 47 |
| | TIMEOUT_INIT | s | 10 |
| "Prüfdruckaufbau für Dichtigkeitsprüfung" | SNKOMPRESSOR: SPANNUNG | V | 4 |
| | PRUEFDRUCK | mbar | 950 |
| | TIMEOUT_DP | s | 60 |
| "Massenänderung nach Druckaufbau" | DM_BUILD_FALL | mg | -15 |
| | DM_BUILD_RISE | mg | 10 |
| "Massenänderung in Druckhaltephase" | MESSDAUER | s | 15 |
| | DM_TEST_MAX | mg | 1,5 |

Beim vorliegenden Test auftretende Fehler und Vorschläge zu ihrer Behebung sind in der folgenden Fehlertabelle (Tabelle 8) wiedergegeben:

Tabelle 8

| Untertest | Fehler |
|---|---|
| "Ausgangsbedingungen herstellen" | TIMEOUT_INIT erreicht<br>→ Ventil 354 öffnet nicht (richtig) oder<br>→ Bypass-Ventil 352 öffnet nicht (richtig) oder<br>→ Offset der Drucksensoren zu groß (Sensoren defekt) |
| "Prüfdruckaufbau für Dichtigkeitsprüfung" | TIMEOUT_DP erreicht<br>→ der Prüfdruck konnte nicht aufgebaut werden<br>　→ Ventil 354 nicht geschlossen/undicht oder<br>　→ Bypass-Ventil 352 nicht geschlossen oder<br>　→ Single-Needle-Ventil 35 nicht geschlossen |
| "Massenänderung nach Druckaufbau" | DM_BUILD_FALL erreicht<br>→ die Massenänderung im System während des Druckaufbaus war zu hoch<br>　→ Ventil 354 undicht oder<br>　→ Single-Needle-Ventil 35 nicht geschlossen |
| | DM_BUILD_RISE erreicht<br>→ die Massenänderung im System während des Druckaufbaus war zu hoch<br>　→ Tank grob undicht oder<br>　→ Füllstand der SN-Kammer zu hoch |
| "Massenänderung in | DM_TEST_MAX überschritten |
| Druckhaltephase" | → Massenänderung im System während des Druckhaltetests zu hoch<br>　→ Tank undicht und/oder<br>　→ SN-Kammer undicht |

Die **fünfte** Ausführungsform betrifft einen Funktionstest des Rückschlagventils 23 im Substituatkanal. Dieser Test kann Bestandteil des oben diskutierten Dichtigkeitstest der ersten Ausführungsform sein, wie zur ersten Ausführungsform beschrieben ist. Geprüft wird, ob das Rückschlagventil 23 im Substituatkanal funktionstüchtig ist. Ein zuvor im EB 1 aufgebauter Druck darf trotz rückwärts laufender Substituatpumpe 17 nicht abfallen. Mittels dieses Tests wird vorteilhaft die Wanderung von Blut in den Substituatkanal und die Kontamination des automatischen Substituatkonnektors 18b (ein Online-Konnektor) mit Blut vermieden.

Bei nicht bestandenem Test kann dieser einmalig wiederholt werden. Eine Behandlung des Patienten ist nur bei bestandenem Test möglich.

Für Details zu diesem auch separat von weiteren Tests durchführbaren Verfahren wird auf dessen oben stehende Beschreibung verwiesen.

Die **sechste** Ausführungsform betrifft einen Funktionstest des Single-Needle(SN)-Pneumatiksystems.

In diesem Test wird geprüft, ob der Single-Needle-Kompressor läuft, ob die Pneumatikventile schalten, die Drucksensoren funktionieren und ob das Pneumatiksystem auf der Maschinenseite dicht ist.

Hierzu wird bei eingelegtem und verpresstem Disposable, und ggf. nach erfolgreich absolviertem T1-Test, ein Druckgradient im Single-Needle-System aufgebaut. Es wird geprüft, ob der Druckgradient innerhalb einer vorgegebenen Zeit gehalten wird oder zumindest nicht mehr als erlaubt abfällt.

Sollte dieser Test nicht bestanden werden, so ist in bestimmten erfindungsgemäßen Ausführungsformen vorgesehen, einen Single-Needle-Betrieb zu unterbinden.

Tritt während des Tests einer beliebigen erfindungsgemäßen Ausführungsform ein Blut-Alarm auf, so wird der laufende Testschritt abgebrochen. Nach Beseitigung des Alarms ist in manchen erfindungsgemäßen Ausführungsformen vorgesehen, dass der abgebrochene Test selbsttätig wiederholt wird.

Steht eine Fehlermeldung des Testschritts an, wenn der Alarm auftritt, so wird diese in einigen erfindungsgemäßen Ausführungsformen automatisch gelöscht, wenn der Testschritt wiederholt wird.

Steht kein Substituat zur Verfügung (Leitfähigkeits-/Temperaturalarm), so wird in bestimmten Ausführungsformen der Test oder Teiltest ebenfalls abgebrochen und anschließend erneut getriggert.

Ist die Leitfähigkeit LF außerhalb eines Toleranzbereichs, so wird in einigen erfindungsgemäßen Ausführungsformen kein Substituat zur Verfügung gestellt.

Fällt der Pegel in der venösen Kammer während eines Tests ab, so wird in bestimmten erfindungsgemäßen Ausführungsformen der Test abgebrochen. Eine Ausnahme hiervon kann der Dichtigkeitstest für die Substituatkonnektion bilden.

Ist einer der Funktionstest nicht bestanden, so wird für den Fall, dass ein arterieller optischer Detektor (OD) dunkel" (= Blut gelangt in den extrakorporalen Blutkreislauf) anzeigt, ein bestätigbarer Alarm erzeugt. Zeigt ein im venösen Abschnitt angeordneter OD (venös) dunkel" an, ist ein Abrüsten des Disposables vorgesehen.

Die Tests und Untertests der oben dargestellten Ausführungsformen können nach Belieben miteinander verbunden werden, sofern und soweit der Fachmann dies nicht als technisch unmöglich erkennt.

Um sicherzustellen, dass die jeweils relevanten Funktionstests vor Beginn jeder Behandlung durchgeführt und Fehler erkannt werden, wird ein entsprechend programmierter oder konfigurierter Funktionstestmonitor eingesetzt. Dieser überwacht die Durchführung der Funktionstests und prüft deren Ergebnisse.

Das erfindungsgemäße Verfahren umfasst in manchen erfindungsgemäßen Ausführungsformen neben den eigentlichen - z. B. wie oben beschriebenen - Funktionstests auch die Bestimmung des Systemoffsets der Fisteldrucksensoren.

Wird beim Übergang des arteriellen optischen Detektors (OD) auf dunkel" (= Blut gelangt in den extrakorporalen Blutkreislauf) erkannt, dass ein Funktionstest nicht oder nicht erfolgreich durchgeführt wurde, so wird in einigen erfindungsgemäßen Ausführungsformen ein entsprechender Blut-Alarm ausgegeben. Dieser kann ggf. zurücksetzbar sein, um etwaige kurzzeitige Störungen des arteriellen ODs durch Luftblasen oder ähnliches überbrücken zu können. Dies ist allerdings nicht stets vorgesehen. Die Überbrückungsdauer kann beispielsweise 0,25 s betragen. Bleibt der arterielle OD dunkel, ist in bestimmten erfindungsgemäßen Ausführungsformen keine Behandlung möglich.

Zusätzlich wird, falls ein Funktionstest nicht bestanden wurde, wenn der venöse OD Blut erkennt, ein nicht zu bestätigender Blut-Alarm erzeugt, der zum Abrüsten des Systems zwingt.

Abweichend hiervon kann insbesondere das Vorgehen bei der Überwachung des SN-System-Funktionstests sein. Wird dieser beim Übergang des venösen ODs auf "dunkel" als nicht bestanden (oder als nicht ausgeführt) erkannt, so wird eine SN-Behandlung durch Setzen einer entsprechenden Variable (z. B. E2_SN_Enable auf SECURE_FALSE) unterbunden. Beim Übergang des arteriellen ODs auf "dunkel" findet in einigen erfindungsgemäßen Ausführungsformen keine Überprüfung statt.

Beim Auftreten von Testfehlern können Status- und Fehlervariablen gesetzt werden. Unabhängig hiervon können Einträge in Fehlertabellen oder -protokollen erzeugt werden. In beiden Fällen können der jeweils durchgeführte Test sowie die Fehlerbedingung, insbesondere auslesbar, hinterlegt sein.

Sollten die Funktionstests bei Erreichen des Soll-Spülvolumens nicht abgeschlossen sein, so wird das Soll-Spülvolumen in einigen erfindungsgemäßen Ausführungsformen automatisch um 50 ml erhöht. Eine entsprechende Meldung kann ausgegeben werden.

Bei Bestehen und/oder bei Nichtbestehen von Funktionstests können entsprechende Meldungen ausgegeben werden.

**Bezugszeichenliste**

| | |
|---|---|
| 1 | extrakorporaler Blutkreislauf |
| 2 | Blutkassette |
| 3 | erste Fluidführung des automatischen Substituatkonnektors 18b |
| 4 | Behandlungsvorrichtung |
| 5 | zweite Fluidführung des automatischen Substituatkonnektors 18b |
| 6 | arterielle Patientenschlauchklemme |
| 7 | venöse Patientenschlauchklemme |
| 8 | arterieller Patientenschlauch oder arterielle Blutleitung |
| 9 | venöser Patientenschlauch oder venöse Blutleitung |
| 10 | dritte Fluidführung des automatischen Substituatkonnektors 18b |
| 11 | Blutpumpe |
| 11' | Bremseinrichtung der Blutpumpe |
| 13 | Zugabestelle für Substituatflüssigkeit (Prädilution) |
| 14 | Zugabestelle für Substituatflüssigkeit (Postdilution) |
| 15 | arterieller Luft-Blut-Detektor |
| 17 | Substituatpumpe |
| 17' | Bremseinrichtung der Substituatpumpe |
| 18a | automatischer Substituatport |
| 18b | automatischer Substituatkonnektor |
| 19 | Blutfilter |
| 19a | Blutkammer |
| 19b | Dialysatkammer |
| 21 | venöse Luftabscheidekammer |
| 23 | Rückschlagventil des Substituatkanals |
| 25 | venöser Luft-Blut-Detektor |
| 26 | Druckluftquelle |
| 27 | Bluteinlass des Blutfilters 19 |
| 28 | Steuer- und/oder Regelungseinrichtung |
| 29 | Blutauslass des Blutfilters 19 |
| 31a | Dialysierflüssigkeitszulaufleitung |
| 31b | Dialysatablaufleitung |
| 33a, b | Drucksensoren |
| 33c | Präfilterdrucksensor |
| 35 | Single-Needle-Ventil |
| 37 | Drucksensor |
| 40 | Hydraulik-Bilanzkammer |
| 41 | Dialysateinlass des Blutfilters 19 |
| 43 | Dialysatauslass des Blutfilters 19 |
| 45 | Drain-Leitung |
| 47 | Sterilluftzuführung |
| V19 | Ventil der Dialysatablaufleitung 31b |
| V24 | Ventil der Dialysierflüssigkeitszulaufleitung 31a |
| V25 | Ventil der Dialysatablaufleitung 31b |
| V28 | Ventil der Drain-Leitung 45 |
| V31 | Ventil der ersten Fluidführung 3 des automatischen Substituatkonnektors 18b |
| V32 | Ventil der zweiten Fluidführung 5 des automatischen Substituatkonnektors 18b |
| V33 | erstes Ventil der Drain-Leitung 45 |
| V42 | Ventil der Sterilluftzuführung 47 |
| V43 | Ventil der dritten Fluidführung 10 des automatischen Substituatkonnektors 18b |

| H33 | Spülport |
| --- | --- |
| P03 | Ultrafiltrationspumpe |
| 100 | Luftverteilerplatte |
| 200 | Pneumatikeinheit |
| 300 | Single-Needle-Unit |
| 301 | Be- und Entlüftung |
| 350 | Single-Needle-Kompressor |
| 352 | Bypass-Ventil |
| 354 | Entlüftungsventil |
| 356 | Drucksensor |
| 358 | Drucksensor der SN-Leitung |
| 360 | Absolutdrucksensor |
| 362 | Temperatursensor im SN-Druckbehälter |
| 370 | Verbinder, verbindet die Single-Needle-Unit 300 mit der Single-Needle-Kammer der Blutkassette 2 |

**Patentansprüche**

1. Verfahren zum Überprüfen wenigstens einer Funktion einer medizinischen Funktionseinrichtung, welche in eine medizinischen Behandlungsvorrichtung (4) eingelegt und mit dieser verbunden und verpresst ist, und/oder einer Funktion dieser Behandlungsvorrichtung (4), wobei zwischen einer Hydraulikeinrichtung oder einer Pneumatikeinheit (200) der Behandlungsvorrichtung (4) und der Funktionseinrichtung wenigstens eine Fluidkommunikation hergestellt ist, wobei das Verfahren die Schritte aufweist:

   - Aufbauen eines Drucks mittels der Hydraulikeinrichtung oder einer Pneumatikeinheit (200) innerhalb der Fluidkommunikation;
   - Messen eines innerhalb der Fluidkommunikation herrschenden Drucks oder einer Druckveränderung;
   - Treffen einer Aussage über die überprüfte Funktion der Funktionseinrichtung aufgrund eines Vergleichs des herrschenden Drucks oder der gemessenen Druckveränderung mit zuvor gespeicherten Werten, Schwellenwerten, Bereichen oder Verläufen;
   - Sperren von Behandlungsmodalitäten und/oder Einschränken von Behandlungsparametern des Behandlungsverfahrens unter Einbezug eines Ergebnisses der Überprüfung wenigstens einer Funktion der medizinischen Funktionseinrichtung und/oder der Behandlungsvorrichtung (4), wobei das Ergebnis mittels des vorliegenden Verfahrens erzielt wurde;
   - Fördern eines vorbestimmten Fördervolumens (MAXFOERDERVOLUMEN) innerhalb der Fluidkommunikation bei rückwärts laufender Substituatpumpe (17) gegen ein Rückschlagventil (23), welches innerhalb eines Substituatkanals vorgesehen ist, und
   - Feststellen einer Druckveränderung innerhalb der Fluidkommunikation,

   wobei das Verfahren mindestens einen der beiden folgenden Schritte aufweist:

   - Feststellen, dass die Substituatpumpe (17) vorwärts fördert oder gefördert hat, falls ein festgestellter Druckanstieg höher als ein maximaler Druckanstieg (MAXDRUCKANSTIEG) oder höher als ein vorbestimmter Betrag einer Druckveränderung (MAXIMALE DRUCKAENDERUNG) ist
   und/oder
   - Feststellen einer Rückschlagfunktion des Rückschlagventils (23), falls ein festgestellter Druckabfall nicht gleich oder höher als ein maximaler Druckabfall (MAXDRUCKABFALL) oder nicht höher als ein vorbestimmter Betrag einer Druckveränderung (MAXIMALE DRUCKAENDERUNG) ist, wobei die medizinische Funktionseinrichtung eine Blutkassette (2) ist, und wobei die Behandlungsvorrichtung (4) eine Blutbehandlungsvorrichtung ist, und wobei das Verfahren automatisch abläuft.

2. Verfahren nach Anspruch 1, wobei der Schritt:

   - Aufbauen eines Drucks mittels der Hydraulikeinrichtung oder einer Pneumatikeinheit (200) innerhalb der Fluidkommunikation

   nur ausgeführt wird, wenn bei einem vorausgehenden Anlegen eines Vordrucks, welcher niedriger als der aufzubauende Druck ist, keine Funktionsabweichung festgestellt wurde.

3. Verfahren nach Anspruch 1 oder 2, mit wenigstens einem der weiteren Schritte:

- Befüllen eines Blutfilters (19), mit welchem die Funktionseinrichtung verbunden ist,- Aufbauen des Drucks mittels einer Substituatpumpe (17) der Behandlungsvorrichtung (4),
- Aufbauen des Drucks gegen die stehende Substituatpumpe (17), und/oder
- Befüllen eines mit der Funktionseinrichtung verbundenen extrakorporalen Blutkreislaufs (1) vor Aufbauen des Drucks.

4. Verfahren nach einem der vorangegangenen Ansprüche, mit den Schritten:

- Feststellen eines Druckabfalls oder eines Druckanstiegs innerhalb der Fluidkommunikation;
- Feststellen einer Durchgängigkeit der Fluidkommunikation oder der Funktionseinrichtung aufgrund eines Vergleichs des festgestellten Druckabfalls oder Druckanstiegs mit zuvor gespeicherten Werten, Schwellenwerten, Bereichen oder Verläufen.

5. Verfahren nach einem der vorangegangenen Ansprüche, mit den Schritten:

- Feststellen eines Druckabfalls oder Druckanstiegs innerhalb der Fluidkommunikation;
- Feststellen einer Dichtigkeit der Fluidkommunikation oder der Funktionseinrichtung aufgrund eines Vergleichs des festgestellten Druckabfalls oder Druckanstiegs mit zuvor gespeicherten Werten, Schwellenwerten, Bereichen oder Verläufen.

6. Verfahren nach einem der vorangegangenen Ansprüche, mit dem Schritt:

- Ermitteln eines aktuellen Druckwertes (WERT_neu) ausgehend von einem bereits vorliegenden Druckwert (WERT_alt), von einem Messwert (MESSWERT) und von einem vorbestimmten Glättungsfaktor (GF) nach der Formel:

$$WERT\_neu = WERT\_alt * GF + MESSWERT* (1 - GF)$$

wobei gilt:

WERT_neu ist ein aktueller Druckwert;
WERT_alt ist der bereits vorliegende Druckwert;
GF ist ein Glättungsfaktor; und
MESSWERT ist ein aktueller Messwert des Drucks.

7. Verfahren nach einem der vorangegangenen Ansprüche, wobei wenigstens eine Funktion, insbesondere das Öffnen, das Schließen oder das Dichten, wenigstens einer der folgenden Komponenten getestet wird:

- Ventil für eine Zugabestelle (13) für Substituat in Prädilution;
- Ventil für eine Zugabestelle (14) für Substituat in Postdilution;
- Single-Needle-Ventil (35);
- Rückschlagventil (23) im Substituatkanal; und/oder
- automatischer Substituatkonnektor (18b).

8. Erfassungseinrichtung, programmiert und/oder konfiguriert zum Durchführen oder Veranlassen eines Verfahrens nach einem der Ansprüche 1 bis 7.

9. Erfassungseinrichtung nach Anspruch 8, aufweisend wenigstens eine Anzeigeeinrichtung zum Anzeigen eines Ergebnisses der Durchführung des Verfahrens nach einem der Ansprüche 1 bis 7 und/oder aufweisend wenigstens eine Alarmeinrichtung zum Ausgeben eines Alarms, wobei die Alarmeinrichtung vorgesehen ist zum Ausgeben eines Alarms für den Fall, dass das Ergebnis der Durchführung des Verfahrens nach einem der Ansprüche 1 bis 7 nicht in einem vorbestimmten Bereich oder Wertebereich liegt.

10. Erfassungseinrichtung nach einem der Ansprüche 8 bis 9, welche programmiert und/oder konfiguriert ist, um derart auf die Behandlungsvorrichtung (4) einzuwirken, dass wenigstens eine Behandlungsoption, zu welcher die überprüfte Funktionseinrichtung konstruktionsbedingt einsetzbar ist, und/oder das Behandeln unter vorbestimmten Behandlungsparametern mittels der konkreten, überprüften Funktionseinrichtung nicht durchführbar ist, falls eine mangelnde Funktionsfähigkeit der Funktionseinrichtung und/oder der Behandlungsvorrichtung (4) oder jeweils einer Komponente hiervon erkannt wurde.

11. Erfassungseinrichtung nach einem der Ansprüche 8 bis 10, welche eine Steuerungsvorrichtung und/oder ein Funktionstestmonitor ist oder jeweils aufweist.

12. Medizinische Behandlungsvorrichtung, welche wenigstens eine Erfassungseinrichtung gemäß einem der Ansprüche 8 bis 11 aufweist und/oder mit dieser in Signalübertragung steht oder zur Signalübertragung verbunden ist.

13. Medizinische Behandlungsvorrichtung nach Anspruch 12, welche als Vorrichtung zur Apherese oder Dialyse, wiederum insbesondere zur Hämodialyse, Hämofiltration oder Hämodiafiltration, ausgestaltet ist.

**Claims**

1. A method for verifying at least one function of a medical functional device which is inserted in and connected with and pressed together with a medical treatment apparatus (4), and/or for verifying at least one function of this treatment apparatus (4), wherein at least one fluid communication is established between a hydraulic device or a pneumatic unit (200) of the treatment apparatus (4) and the functional device, wherein the method comprises the steps of:

   - building up a pressure by means of the hydraulic device or of a pneumatic unit (200) within the fluid communication;
   - measuring a pressure prevailing within the fluid communication or a pressure change;
   - making a statement about the verified function of the functional device based on a comparison of the prevailing pressure or of the measured pressure change with previously saved values, threshold values, ranges or evolutions;
   - blocking treatment modalities and/or restricting treatment parameters of the treatment process taking into account a result of the verifying of at least one function of the medical functional device and/or of the treatment apparatus (4), wherein the result has been obtained by means of the present method;
   - conveying a predetermined feed volume (MAXFOERDERVOLUMEN) within the fluid communication with the substitute pump (17) running in reverse direction against a non-return valve (23) which is provided within a substitute channel, and
   - detecting a pressure change within the fluid communication,

   wherein the method comprises at least one of the two following steps:

   - detecting that the substitute pump (17) conveys or conveyed forwards, if a detected pressure rise is higher than a maximum pressure rise (MAXDRUCKANSTIEG) or higher than a predetermined value of a pressure change (MAXIMALE DRUCKAENDERUNG)
   and/or
   - detecting a non-return function of the non-return valve (23), if a detected pressure drop is not equal to or higher than a maximum pressure drop (MAXDRUCKABFALL) or not higher than a predetermined value of a pressure change (MAXIMALE DRUCKAENDERUNG),

   wherein the medical functional device is a blood cassette (2), and wherein the treatment apparatus (4) is a blood treatment apparatus, and wherein the method runs automatically.

2. The method according to claim 1, wherein the step of:

   - building up a pressure by means of the hydraulic device or a pneumatic unit (200) within the fluid communication is only executed if no function deviation was detected with a previously applied pre-pressure, which is lower than the pressure to be built-up.

3. The method according to claim 1 or 2, comprising at least one of the two further steps of:

- filling a blood filter (19) to which the functional device is connected;
- building up the pressure by means of a substituate pump (17) of the treatment apparatus (4),
- building up the pressure against the stopped substituate pump (17), and/or
- filling an extracorporeal blood circuit (1) which is connected with the functional device before building up the pressure.

4.  The method according to anyone of preceding claims, comprising the steps of:

   - detecting a pressure drop or a pressure rise within the fluid communication;
   - detecting a patency of the fluid communication or of the functional device based on a comparison of the detected pressure drop or pressure rise with previously stored values, threshold values, ranges or evolutions.

5.  The method according to anyone of preceding claims, comprising the steps of:

   - detecting a pressure drop or pressure rise within the fluid communication;
   - detecting a tightness of the fluid communication or of the functional device based on a comparison of the detected pressure drop or pressure rise with previously stored values, threshold values, ranges or evolutions.

6.  The method according to anyone of preceding claims, comprising the step of:

   - determining a current pressure value (WERT_neu) from an already existing pressure value (WERT_alt), from a measured value (MESSWERT) and from a predetermined smoothing factor (GF) according to the formula:

   $$\text{WERT\_neu = WERT\_alt * GF + MESSWERT* (1 - GF)}$$

   whereby:

   WERT_neu is a current pressure value;
   WERT_alt is an already existing pressure value;
   GF is a smoothing factor; and
   MESSWERT is a current measured value of the pressure.

7.  The method according to anyone of preceding claims, wherein at least one function, in particular the opening, the closing or the sealing of at least one of the following components is verified:

   - valve for an addition point (13) for substituate in predilution;
   - valve for an addition point (14) for substituate in postdilution;
   - single-needle valve (35);
   - non-return valve (23) in the substituate channel; and/or
   - automatic substituate connector (18b).

8.  A detecting means programmed and/or configured for executing or prompting a method according to anyone of claims 1 to 7.

9.  The detecting means according to claim 8, comprising at least one display means for displaying a result of the execution of the method according to anyone of claims 1 to 7 and/or comprising at least one alarm means for outputting an alarm, wherein the alarm means is provided for outputting an alarm in the event that the result of the execution of the method according to anyone of claims 1 to 7 does not lie within a predetermined range or value range.

10. The detecting means according anyone of claims 8 to 9 programmed and/or configured to interact with the blood treatment apparatus (4), such that at least one treatment option for which the verified functional device is usable due to its construction and/or the treatment with predetermined treatment parameters by means of the concrete, verified functional device is not executable if a lack of functionality of the functional device and/or of the treatment apparatus (4), or respectively of a component thereof, has been identified.

11. The detecting means according to anyone of claims 8 to 10, which is a control apparatus and/or a function test monitor or comprising such one.

**12.** A medical treatment apparatus comprising at least one detecting means according to anyone of claims 8 to 11 and/or being in signal transmission or being connected for signal transmission with it.

**13.** The medical functional device according to claim 12, embodied as an apparatus for apheresis or dialysis, in turn again in particular for hemodialysis, hemofiltration or hemodiafiltration.

**Revendications**

**1.** Un procédé de contrôle d'au moins une fonction d'un dispositif fonctionnel médical inséré dans un appareil de traitement médical (4) et connecté et maintenu par compression avec celui-ci, et/ou d'au moins une fonction de cet appareil de traitement (4), où au moins une communication fluidique est établie entre un dispositif hydraulique ou une unité pneumatique (200) de l'appareil de traitement (4) et le dispositif fonctionnel, le procédé comprenant les étapes suivantes :

- établir une pression au moyen du dispositif hydraulique ou d'une unité pneumatique (200) à l'intérieur de la communication fluidique;
- mesurer une pression régnant à l'intérieur de la communication fluidique ou un changement de pression;
- donner une indication relative à la fonction contrôlée du dispositif fonctionnel sur la base d'une comparaison de la pression régnant ou du changement de pression mesuré avec des valeurs, des valeurs-seuils, des plages ou des évolutions préalablement enregistrées;
- bloquer des modalités de traitement et/ou restreindre des paramètres de traitement du procédé de traitement en tenant compte d'un résultat du contrôle d'au moins une fonction du dispositif fonctionnel médical et/ou de l'appareil de traitement médical (4), où le résultat a été obtenu au moyen du présent procédé;
- acheminer un volume d'acheminement prédéterminé (MAXFOERDERVOLUMEN) à l'intérieur de la communication fluidique avec la pompe de substitut (17) fonctionnant en sens inverse contre un clapet anti-retour (23) prévu à l'intérieur d'un conduit du substitut, et
- constater un changement de pression à l'intérieur de la communication fluidique,

où le procédé comprend au moins une des deux étapes suivantes:

- constater que la pompe de substitut (17) achemine ou a acheminé vers l'avant, dans le cas où une augmentation de pression constatée est supérieure à une augmentation de pression maximale (MAXDRUCKANSTIEG) ou supérieure à une valeur prédéterminée d'un changement de pression (MAXIMALE DRUCKAENDERUNG) et/ou
- constater une fonction anti-retour du clapet anti-retour (23), dans le cas où une chute de pression constatée n'est pas égale ou supérieure à une chute de tension maximale (MAXDRUCKABFALL) ou n'est pas supérieure à une valeur prédéterminée d'un changement de pression (MAXIMALE DRUCKAENDERUNG),

où le dispositif fonctionnel médical est une cassette à sang (2), et où l'appareil de traitement (4) est un appareil de traitement du sang, et où le procédé est exécuté automatiquement.

**2.** Un procédé selon la première revendication, où l'étape suivants :

- exercer une pression au moyen d'un dispositif hydraulique ou d'une unité pneumatique (200) à l'intérieur de la communication fluidique

n'est exécutée que si aucune anomalie de fonctionnement n'a été constatée lors de l'application préalable d'une pré-pression inférieure à la pression à établir.

**3.** Un procédé selon la première ou la seconde revendication, comprenant au moins une des étapes suivantes :

- remplir un filtre sanguin (19) avec lequel le dispositif fonctionnel est connecté,
- établir la pression au moyen d'une pompe de substitut (17) de l'appareil de traitement (4),
- établir la pression contre la pompe de substitut (17) à l'arrêt, et/ou
- remplir un circuit sanguin extracorporel (1) connecté au dispositif fonctionnel avant d'établir la pression.

**4.** Un procédé selon l'une quelconques des revendications précédentes comprenant les étapes suivantes :

- constater une chute de pression ou une augmentation de pression à l'intérieur de la communication fluidique;
- constater, sur la base d'une comparaison de la chute ou de l'augmentation de pression constatée avec des valeurs, des valeurs-seuils, des plages ou des évolutions préalablement enregistrées, que la communication fluidique ou le dispositif fonctionnel n'est pas obstrué(e).

**5.** Un procédé selon l'une quelconque des revendications précédentes comprenant les étapes suivantes :

- constater une chute ou une augmentation de pression à l'intérieur de la communication fluidique;
- constater une étanchéité de la communication fluidique ou du dispositif fonctionnel sur la base d'une comparaison de la chute ou de l'augmentation de pression constatée avec des valeurs, des valeurs-seuils, des plages ou des évolutions préalablement enregistrées.

**6.** Un procédé selon l'une des revendications précédentes comprenant l'étape :

- déterminer une valeur de pression actuelle (WERT_neu) à partir d'une valeur de pression (WERT_alt) déjà existante, d'une mesure (MESSWERT) et d'un facteur de lissage (GF) prédéfini selon la formule suivante :

$$WERT\_neu = WERT\_alt * GF + MESSWERT * (1 - GF)$$

dans laquelle:

WERT_neu est une valeur de pression actuelle;
WERT_alt est la valeur de pression déjà existante;
GF est un facteur de lissage; et
MESSWERT est une mesure actuelle de la pression.

**7.** Un procédé selon l'une quelconque des revendications précédentes, où au moins une fonction, notamment l'ouverture, la fermeture ou l'étanchéité d'au moins un des composants suivants est testés :

- vanne pour un point d'addition (13) de substitut en pré-dilution;
- vanne pour un point d'addition (14) de substitut en post-dilution;
- vanne « single-needle » (35);
- clapet anti-retour (23) dans le conduit du substitut; et/ou
- connecteur automatique de substitut (18b).

**8.** Un dispositif de détection programmé et/ou configuré pour exécuter ou déclencher un procédé selon l'une des revendications 1 à 7.

**9.** Un dispositif de détection selon la revendication 8, comprenant au moins un dispositif d'affichage pour afficher un résultat de l'exécution du procédé selon l'une des revendications 1 à 7 et/ou comprenant au moins un système d'alarme pour émettre une alarme, où le système d'alarme est prévu pour émettre une alarme dans le cas où le résultat de l'exécution du procédé selon l'une des revendications 1 à 7 ne se situe pas dans une plage prédéterminée ou dans un intervalle de valeurs prédéterminé.

**10.** Un dispositif de détection selon l'une des revendications 8 à 9, programmé et/ou configuré pour agir sur l'appareil de traitement (4) de telle sorte qu'au moins une option de traitement pour laquelle le dispositif fonctionnel contrôlé peut être utilisé, de par sa construction, et/ou le traitement selon les paramètres de traitement prédéterminés au moyen du dispositif fonctionnel concret contrôlé, n'est pas exécutable, dans le cas où un dysfonctionnement du dispositif fonctionnel et/ou de l'appareil de traitement (4), ou respectivement d'un des composants de celui-ci, serait détecté.

**11.** Un dispositif de détection selon l'une des revendications 8 à 10, étant un moyen de commande et/ou un moniteur de test de fonctionnement ou comprenant ceux-ci.

**12.** Un appareil de traitement médical comprenant au moins un dispositif de détection selon l'une des revendications 8 à 11 et/ou étant en transmission de signal avec celui-ci ou étant connecté avec celui-ci pour la transmission de signal.

**13.** Un appareil de traitement médical selon la revendication 12, conçu comme un appareil pour l'aphérèse ou la dialyse, et plus particulièrement pour l'hémodialyse, l'hémofiltration ou l'hémodiafiltration.

Fig. 1

Fig 2

EP 2 736 558 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20040223857 A1
- DE 102207042964 A1
- WO 2010121819 A